# FASCICULE DE BREVET EUROPEEN

(11) **EP 0 971 915 B1**
(45) Date de publication et mention de la délivrance du brevet: **14.05.2003**
(21) Numéro de dépôt: 98913841.7
(22) Date de dépôt: 05.03.1998
(51) Int. Cl.: C07D 333/20

(54) **PROCEDE DE PREPARATION DE DERIVES DE 2-THIENYLETHYLAMINE**
VERFAHREN ZUR HERSTELLUNG VON 2-THIENYLAMIN-DERIVATEN
METHOD FOR PREPARING 2-THIENYLETHYLAMINE DERIVATIVES

(30) Priorité: 05.03.1997 FR 9702621
(43) Date de publication de la demande: 19.01.2000
(73) Titulaire: SANOFI-SYNTHELABO, 75013 Paris (FR)
(72) Inventeur: CASTRO, Bertrand, F-34130 Saint Aunes (FR); DORMOY, Jean-Robert, F-04200 Sisteron (FR); PREVIERO, Aldo, I-35040 Masi (IT)
(74) Mandataire: Bernasconi, Jean Raymond
(86) Numéro de dépôt international: FR9800441
(87) Numéro de publication internationale: WO98039322

(56) Documents cités:
- EP-A- 0 321 349
- EP-A- 0 355 710
- EP-A- 0 465 358
- EP-A- 0 466 569
- M.E. DULLAGHAN ET AL.: "Studies on the chemistry of heterocyclics. The Darzens reaction" JOURNAL OF ORGANIC CHEMISTRY, vol. 17, 1952, EASTON US, pages 1183-1186, XP002046377
- C.L. BROWNE ET AL.: "Isomeric amino alcohols from the reaction of styrene oxide with benzylamine" JOURNAL OF ORGANIC CHEMISTRY, vol. 17, 1952, EASTON US, pages 1187-1193, XP002046378

## Description

La présente invention se rapporte, d'une manière générale, à un nouveau procédé de préparation de dérivés de 2-thiényl-éthylamine.

En particulier, l'invention concerne un nouveau procédé pour la préparation de dérivés N-phénylacétiques de 2-thiényl-éthylamine de formule générale : 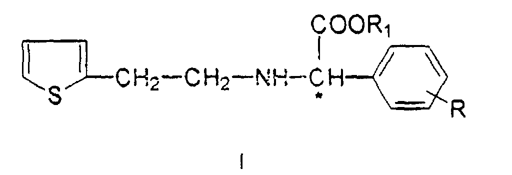 ainsi que de leurs sels d'addition d'acide, dans laquelle R représente un atome d'halogène tel que chlore ou brome et R₁ représente un groupement alkyle en C₁-C₄, de préférence méthyle.

Ces composés de formule 1 possèdent un carbone asymétrique représenté par l'astérisque et, en conséquence, peuvent se présenter sous la forme de mélange racémique ou d'isomères optiques individuels (-)-R et (+)-(S).

Ainsi, l'invention se rapporte à la préparation de dérivés de 2-thiényl-éthylamine, de formule I qu'ils soient sous la forme de mélange racémique ou d'énantionnères dextrogyre ou lévogyre individuels.

Dans la formule I ci-dessus, le groupement R peut se trouver en position ortho, méta ou para du groupe acétate de préférence en position ortho. Par ailleurs, le chlore représente un groupement R préféré.

En conséquence et selon un aspect préférentiel, l'invention concerne un procédé pour la préparation de l'énantiomère (+)-(S) des composés de formule I, en particulier l'énantiomère (+)-(S) de l'α-(2-thiényl-2-éthylamino)-α-(2-chloro-phényl)-acétate de méthyle.

Ces composés de formule I sont des produits connus et peuvent être utilisés pour la préparation de composés pharmacologiquement actifs.

Par exemple, le (+)-(S)-α-(2-thiényl-2-éthylamino)-α-(2-chloro-phényl)-acétate de méthyle a été décrit dans le brevet EP 466569 ainsi que son utilisation pour la préparation du (+)-(S)-α-(4,5,6,7-tétrahydro-thiéno[3,2-c]-5-pyridyl)-α-(2-chloro-phényl)-acétate de méthyle ou clopidogrel.

Cet énantiomère de formule développée : 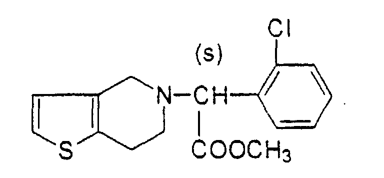 est connu pour son intérêt en thérapeutique notamment pour ses activités antiagrégante plaquettaire et antithrombotique.

On a décrit dans ce brevet EP 466569 un procédé pour la préparation du clopidogrel via un composé racémique, à savoir le (R,S)-α-(4,5,6,7-tétrahydrothiéno [3,2-c-]-5-pyridyl)-α-(2-chloro-phényl)-acétate de méthyle que l'on soumet à un processus de résolution.

Selon ce processus, on cristallise sélectivement un sel diastéréoisomérique de ce composé racémique avec l'acide (-)-(R)-10-campho-sulfonique, ce qui conduit au camphosulfonate chiral de clopidogrel et on en libère ensuite la base par déplacement de l'acide (-)-(R)-10-campho-sulfonique.

Cette voie d'accès, bien que d'utilisation classique pour préparer un composé chiral, peut être considérée comme peu pratique au plan économique puisqu'elle nécessite à la fois le recyclage de l'isomère non désiré et celui du sel de l'acide chiral (-)-(R)-10-campho-sulfonique employé à la résolution.

Une méthode plus convergente pour la préparation notamment du clopidogrel, a été proposée dans le brevet EP 466569, méthode selon laquelle on traite, dans une première étape, le (+)-2-chloro-phénylglycinate de méthyle avec un halogénure ou sulfonate de 2-thiényl-éthyle et ce, dans un solvant. durant plusieurs heures, à une température comprise entre 50°C et 100°C et en présence d'une base, pour donner après salification, le chlorhydrate du (+)-(S)-α-(2-thiényl-2-éthylamino)-α-(2-chloro-phényl)-acétate de méthyle avec un rendement d'environ 50%.

Toutefois, on a remarqué que le choix du solvant, dans cette méthode, n'est pas indifférent si l'on veut obtenir un seul des énantiomères du α-(2-thiényl-2-éthylamino)-α-(2-chloro-phényl)-acétate de méthyle par réaction d'un des énantiomères du 2-chloro-phénylglycinate de méthyle, une racémisation partielle se produisant dans certains solvants.

Par ailleurs, ce procédé présente l'inconvénient de nécessiter un temps de contact assez prolongé à une température supérieure à l'ambiante, par exemple pendant 40 heures à 80°C, pour l'obtention du (+)-(S)-α-(2-thiényl-2-éthylamino)-α-(2-chloro-phényl)-acétate de méthyle, ces conditions opératoires ne pouvant qu'influencer de manière défavorable le prix de revient du produit final.

Enfin, les rendements en dérivés de 2-éthyl-éthylamine de formule I ainsi obtenus restent relativement modestes puisque de l'ordre de 50%.

La recherche d'un procédé industriel pour la préparation des dérivés de 2-thiényl-éthylamine de formule I qu'ils soient sous forme racémique ou d'énantiomères individuels, mettant en oeuvre des intermédiaires de synthèse selon un processus opératoire peu onéreux et procurant un rendement satisfaisant en produit souhaité reste d'un intérêt incontestable.

Or, on a maintenant trouvé, de manière surprenante, qu'il est possible d'obtenir les dérivés de 2-thiényl-éthylamine de formule I notamment leurs énantiomères dextrogyres, en évitant les inconvénients rapportés précédemment et selon des rendements supérieurs à ceux obtenus avec le procédé antérieur puisque l'on enregistre la formation d'au moins 90% de ces composés sous forme de base libre par rapport au rendement théorique.

Ainsi, selon l'invention, on prépare les dérivés de 2-thiényl-éthylamine de formule I en faisant réagir un dérivé thiénylglycidique de formule générale : 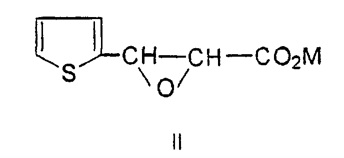 dans laquelle M représente un atome de métal alcalin tel que lithium, potassium ou de préférence sodium ou une fraction d'un atome de métal alcalino-terreux tel que calcium ½ ou magnésium ½, avec un ester de phénylglycine de formule générale : 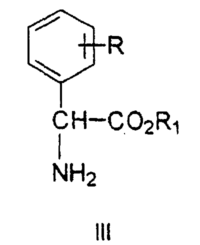 dans laquelle R et R₁ ont la même signification que précédemment pour I. éventuellement sous forme d'un sel d'acide fort, par exemple le chlorhydrate ou le méthanesulfonate, en présence d'un borohydrure de métal alcalin de formule générale :

X-Y IV

dans laquelle X représente un atome de métal alcalin de préférence sodium et Y représente un groupement de formule :

-BH₃CN ou -BH_{(4-w)}Z_{w}

dans laquelle Z représente un reste d'acide carboxylique, généralement un reste de formule générale :

R₂-CO₂-

dans laquelle R₂ représente alkyle en C₁-C₁₀ par exemple méthyle, et w représente 1,2 ou 3, ce qui fournit le composé désiré sous forme de base libre que l'on peut faire réagir, si nécessaire, avec un acide pour obtenir un sel d'addition de ce composé.

Le composé de formule III peut être sous forme racémique ou, au contraire, sous forme d'énantiomères (+)-(S) ou (-)-(R).

En fait, on a pu mettre en évidence que le procédé de l'invention s'effectue avec rétention de configuration lorsque l'ester de phénylglycine de formule III est sous forme d'énantiomère dextrogyre ou lévogyre séparé, la pureté optique de l'énantiomère du composé de formule I dépendant exclusivement de la pureté optique de l'énantiomère du composé de départ de formule III.

En raison de cette stéréospécificité du procédé de l'invention, les esters de formule III sous forme d'énantiomères individuels, en particulier (+)-(S)-α-amino-α-(2-chloro-phényl)-acétate de méthyle, peuvent être considérés comme préférés.

Lorsque le borohydrure de métal alcalin correspond à un cyanoborohydrure c'est-à-dire un composé de formule IV dans laquelle Y représente le groupement -BH₃ CN, le procédé de l'invention se déroule avantageusement et préférentiellement en présence d'un acide faible tel qu'un acide alkylcarboxylique en C₁-C₄, par exemple l'acide acétique et de préférence à une concentration n'excédant pas 0,50 mole/l.

A titre indicatif, on a enregistré un rendement maximal en composé de formule I à une concentration de 0,30 à 0,35 mole/l en acide faible tel que l'acide acétique dans le solvant organique utilisé par exemple le méthanol.

De même, lorsque le composé de formule IV est un cyanoborohydrure alcalin, on utilise :
a) un sel d'acide fort du composé de formule III en particulier d'un composé de formule III dans laquelle R représente un atome d'halogène en position ortho, en général un sel d'acide fort d'un dérivé 2-chloro-phényle de formule III et plus particulièrement un sel d'acide fort de l'α-amino-α-(2-chloro-phényl)-acétate de méthyle,
b) le dérivé thiényle de formule II de préférence en léger excès par rapport au composé de formule III, jusqu'à 0,5 mole en surplus par mole de composé de formule III.

De même, lorsque le borohydrure de métal alcalin correspond à un acyloxyborohydrure c'est-à-dire un composé de formule IV dans laquelle Y représente le groupement -BH_{(4-w)}Z_{w}, le procédé de l'invention est mis en oeuvre de préférence dans un acide faible comme solvant tel qu'un acide alkylcarboxylique en C₁-C₄, par exemple l'acide acétique en l'absence d'autre solvant.

Toutefois, on peut également utiliser un milieu réactionnel formé d'un solvant organique tel que de type alcool par exemple le méthanol ou un hydrocarbure halogéné ou non tel que le benzène, le toluène ou un xylène ou encore le dichlorométhane, et d'un acide faible tel que ci-dessus. Dans ce cas, l'acide faible en question est présent, préférentiellement, à raison d'au moins 50% en volume par rapport au solvant organique.

Par ailleurs lorsque le composé de formule IV est un acyloxyborohydrure alcalin, on utilise généralement des quantités équimoléculaires de ce composé de formule IV et d'ester de phénylglycine de formule III, ce composé de formule III étant mis en oeuvre de préférence en excès, c'est-à-dire jusqu'à 2,2 équivalents molaires par équivalent molaire de dérivé thiénylglycidique de formule II.

Quant aux borohydrures de métal alcalin de formule IV, ils comprennent notamment des cyanoborohydrures de métal alcalin, de préférence le cyanoborohydrure de sodium (NaBH₃CN) mais également des borohydrures de restes d'acides carboxyliques. Ceux-ci peuvent être obtenus extemporanément dans un solvant approprié tel que le dichlorométhane par mélange d'un borohydrure de formule générale :

XBH₄ VI

dans laquelle X a la même signification que précédemment, par exemple sodium, avec un acide carboxylique de formule générale :

R₂-CO₂H VII

dans laquelle R₂ a la même signification que précédemment.

Selon des mises en oeuvre habituelles, on prépare les borohydrures de formule IV dans laquelle Y représente un groupement-BH_{(4-w)}Z_{w}
soit
a) par addition lente et sous agitation d'un borohydrure de métal alcalin de formule XBH₄ dans laquelle X a la même signification que précédemment, par exemple le borohydrure de sodium, à un acide de formule VII, qui peut être en excès stoechiométrique, par exemple l'acide acétique, cet acide étant refroidi à une température inférieure à la température ambiante.
soit
b) par addition lente et sous agitation de 1 à 3 équivalents molaires d'acide de formule VII à une suspension de borohydrure de métal alcalin de formule XBH₄ telle que ci-dessus et ce, dans le solvant organique choisi, par exemple le dichlorométhane.
   Après élimination du solvant, le résidu repris dans un acide de formule VII peut constituer un milieu réactionnel approprié pour la mise en oeuvre du procédé de l'invention.

Les borohydrures de formule IV utilisés dans le procédé de l'invention sont généralement mis en oeuvre à une concentration qui n'excède pas 0,40 mole/l, celle-ci correspondant sensiblement à la concentration saturante, c'est-à-dire la concentration au delà de laquelle le rendement de la réaction n'augmente plus.

Les dérivés thiényl-glycidiques de formule II peuvent être préparés selon des méthodes connues.

Par exemple, on peut les obtenir par application du procédé proposé dans le brevet EP 465358 reposant sur une réaction de Darzens.

Selon cette méthode, on fait réagir dans l'isopropanol et à température ambiante, le 2-thiényl-carboxaldehyde avec un haloacétate d'isopropyle, par exemple le chloroacétate d'isopropyle en présence d'un isopropylate de métal alcalin, préférentiellement l'isopropylate de sodium, ce qui fournit le 2-thiénylglycidate d'isopropyle que l'on saponifie ensuite au moyen d'un hydroxyde de métal alcalin ou alcalino-terreux, pour obtenir finalement l'ester glycidique ou glycidate désiré de formule II.

Plus généralement, cette méthode peut être mise en oeuvre à partir d'un haloacétate de méthyle tel que le chloroacétate de méthyle, la réaction se déroulant dans un alcanol en C₁-C₄, par exemple le méthanol.

Quant aux esters de glycine de formule III, il s'agit également de composés connus ou pouvant être préparés par des méthodes connues, qu'ils soient sous forme (-)-(R), (+)-(S) ou sous forme racémique.

A cet effet, on peut utiliser la méthode décrite dans le brevet EP 466569 selon laquelle on estérifie l'aminoacide racémique correspondant ou ses énantiomères individuels, par réaction avec le chlorure de thionyle et un alcanol en C₁-C₄.

De même, les sels d'acide fort des énantiomères des esters de formule III, peuvent être obtenus également par recristallisation du sel formé par le racémate du même composé de formule III avec un acide optiquement actif tel que les acide (+) ou (-) tartrique dans l'isopropanol ou encore les acides (+) ou (-)-10-campho-sulfonique dans l'acétone en présence ou non de méthyléthylcétone, puis par traitement avec un acide fort approprié pour obtenir le sel désiré.

De manière alternative, on peut préparer les énantiomères individuels des esters de formule III sous forme de sel d'acide fort au départ de l'énantiomère de configuration opposée dudit ester, éventuellement en mélange avec l'énantiomère désiré dudit ester, cet ester de départ étant sous forme de base ou de sel d'addition d'acide faible, par exemple sous forme d'acétate.

Selon ce procédé, on traite l'énantiomère ou le mélange d'énantiomères de départ éventuellement en présence d'un cosolvant polaire ou apolaire tel que l'isopropanol, ou un mélange de tels cosolvants, avec un composé cétonique, de préférence l'acétone et avec un acide α-aminé N- protégé à savoir la N-(2,4-dinitrobenzoyl)-phénylglycine sous forme d'un énantiomère, le traitement ayant lieu en présence d'un acide carboxylique de préférence l'acide acétique, de manière à induire une racémisation totale et la précipitation concomitante d'un sel diastéréoisomérique de l'ester de formule III et de la N-(2,4-dinitrobenzoyl)-phénylglycine. On hydrolyse, par la suite, en présence d'un acide fort tel que l'acide chlorhydrique, le sel diastéréoisomérique en question pour obtenir l'énantiomère désiré de formule III sous forme de sel d'acide fort.

Les exemples non limitatifs suivants illustrent le procédé de l'invention.

### PREPARATIONS

### a) 2-Thiénylglycidate de sodium

Dans un flacon de 250 ml, on introduit 100ml de chlorure de méthylène, 8,3 ml de 2-thiényl-carboxaldéhyde à 98% et 9,3 ml de chloroacétate de méthyle puis on homogénéise par agitation magnétique.

On place le mélange dans un bain à 0°C (glace/eau) puis on ajoute lentement, en une heure, 18 ml de méthylate de sodium à 30%. On poursuit l'agitation pendant 2 heures à froid et on laisse ensuite revenir à température ambiante. On ajoute ensuite, au milieu réactionnel, 50 g de glaçons et on agite jusqu'à dissolution totale. On transvase le milieu dans une ampoule à décanter et on sépare les deux phases. On lave la phase organique avec 50 ml d'acide chlorhydrique 0,5 N puis une fois à l'eau distillée. On sèche sur sulfate de sodium anhydre et on filtre. On concentre sous vide à une température inférieure à 30°C puis on laisse revenir à 0°C dans un bain de glace. On verse alors, sur le résidu huileux, 50 ml d'éthanol absolu et 16 ml de méthylate de sodium à 30% puis on ajoute lentement 1,6 ml d'eau distillée ce qui provoque la formation immédiate d'un précipité. On place ce précipité en chambre froide durant 12 heures puis on l'essore, on le lave avec de l'éthanol absolu puis avec de l'éther éthylique. On sèche ensuite dans un dessicateur.

De cette manière, on obtient 14,6 g de 2-thiénylglycidate de sodium.

### b) Acide (R,S)-α-amino-α-(2-chloro-phényl)-acétique

Dans un flacon de 500 ml, on introduit 6,88 g de chlorure d'ammonium, 11,64 g de cyanure de potassium et 200 ml d'ammoniaque à 30%. Sous agitation magnétique, on ajoute alors 200 ml de méthanol contenant 11,2 ml de 2-chlorobenzaldehyde à 99 % puis, sous agitation occasionnelle, on porte le milieu réactionnel à 45°C pendant une heure. On dilue alors avec 200 ml d'eau distillée et on extrait deux fois avec 200 ml d'acétate d'éthyle.

On réunit les phases organiques puis on les lave à l'eau distillée. On sèche sur sulfate de sodium anhydre, on filtre et on évapore à sec, ce qui fournit un résidu huileux.

On hydrolyse alors ce résidu par ajout de 200 ml d'acide chlorhydrique 6N, on porte au reflux et on abandonne le mélange durant 4 heures minimum. On lave l'hydrolysat avec du chloroforme jusqu'a disparition de la couleur jaune due à l'excès de réactif puis on évapore l'excès d'acide chlorhydrique en réduisant le volume de moitié par évaporation sous vide. On ajoute 100 ml d'eau distillée chaude et on ajuste le pH à 5,27 avec de l'ammoniaque, ce qui provoque un début de précipitation d'acide (R,S)-a-amino-α-(2-chloro-phényl)-acétique.

On abandonne alors le milieu en chambre froide durant 12 heures, on essore et on sèche sous vide en présence d'anhydride phosphorique, pour obtenir 8,45 g d'acide (R,S)-α-amino-α-(2-chloro-phényl)-acétique sous formé de cristaux blancs.

### c) Chlorhydrate du (R,S)-α-amino-α-(2-chloro-phényl)-acétate de méthyle.

Sous agitation magnétique et dans un bain de glace, on additionne lentement 5 ml de chlorure de thionyle dans 100 ml de méthanol. On dissout alors, dans ce mélange, les cristaux d'acide (R,S)-α-amino-α-(2-chlorophényl)-acétique obtenus précédemment et on porte le milieu réactionnel à 40°C. On laisse la réaction se poursuivre pendant 48 heures à 40°C puis on évapore à sec sous vide. On dissout le résidu dans du méthanol et on évapore à nouveau sous pression réduite. On ajoute ensuite 200 à 300 ml d'éther éthylique et on abandonne le milieu durant 15 heures à 5 à 6°C, ce qui provoque la formation de cristaux. On essore ces cristaux, on les lave à l'éther éthylique et on les sèche sous vide.

De cette manière, on obtient 9,5 g de chlorhydrate de (R,S)-α-amino-α-(2-chloro-phényl)-acétate de méthyle.

### d) (-)-(2,4-Dinitrobenzoyl)-phénylglycinate de (+)-(S)-α-amino-α-(2-chlorophényl)-acétate de méthyle.

On réalise une suspension de 7,1 g de chlorhydrate de (R,S)-α-amino-α-(2-chloro-phényl)-acétate de méthyle dans 50 ml d'acétate d'éthyle, on y ajoute 50 ml d'ammoniaque 2 à 3 molaires et on extrait. On sépare la phase organique et on la sèche sur sulfate de sodium anhydre.

On filtre directement dans un récipient de 500 ml puis on évapore à sec à une température inférieure à 40°C.

Sous agitation magnétique, on ajoute alors 45 ml d'isopropanol et 10 g de (-)-(2,4-dinitrobenzoyl)-phénylglycine préalablement solubilisée dans 60 ml d'acétone contenant 5% d'acide acétique. On ensemence alors avec quelques germes du composé désiré, on ajoute 120 ml d'hexane et on maintient l'agitation durant 15 minutes. On abandonne le milieu durant 18 heures à 35°C puis durant 24 heures à 5 à 6°C, ce qui provoque la formation de cristaux que l'on essore, rince avec un mélange acétone/hexane vol./vol. et sèche.

De cette manière, on obtient 9,5 g de (-)-(2,4-dinitrobenzoyl)-phénylglycinate de (+)-(S)-α-amino-α-(2-chloro-phényl)-acétate de méthyle. Pureté optique : 98% (chromatographie phase gazeuse)

### e) Chlorhydrate du (+)-(S)-α-amino-α-(2-chloro-phényl)-acétate de méthyle

On introduit 3 g de (-)-(2,4-dinitrobenzoyl)-phénylglycinate de (+)-(S)-α-amino-α-(2-chloro-phényl)-acétate de méthyle dans 20 ml d'une solution 1M en carbonate sodique et on extrait deux fois avec 10 ml d'acétate d'éthyle.

On sèche sur sulfate de sodium anhydre les phases organiques regroupées, on traite avec 10 ml d'acide chlorhydrique 1N dans le méthanol et on concentre sous vide. On dissout le résidu dans la quantité minimale de méthanol et on provoque la cristallisation par ajout d'éther diéthylique.

De cette manière, on recueille le chlorhydrate de (+)-(S)-α-amino-α-(2-chloro-phényl)-acétate de méthyle avec un rendement pratiquement quantitatif.

### f) (+)-(S)-α-amino-α-(2-chloro-phényl)-acétate de méthyle

On introduit 2,36 g (10⁻² mole) de chlorhydrate de (+)-(S)-α-amino-α-(2-chloro-phényl)-acétate de méthyle dans 50 ml d'acétate d'éthyle ou de chlorure de méthylène et on ajoute 20 ml d'ammoniaque 1,5 M ou de bicarbonate de sodium à 5 %. On agite, décante la phase organique et extrait la phase aqueuse avec 10 ml d'acétate d'éthyle. On rassemble les phases organiques, traite avec du sulfate de sodium anhydre et concentre à pression réduite jusqu'à absence de distillat. On reprend ensuite le résidu formé de (+)-(S)-α-amino-α-(2-chloro-phényl)-acétate de méthyle dans 50 ml d'acide acétique.

### EXEMPLE 1

### Chlorhydrate du (+)-(S)-α-(2-thiényl-2-éthylamino)-α-(2-chloro-phényl)-acétate de méthyle

Dans un flacon de 100 ml, on place 1,92 g (10⁻² mole) de 2-thiénylglycidate de sodium, 2,36 g de chlorhydrate de (+)-(S)-α-amino-α-(2-chloro-phényl)-acétate de méthyle et 0,63 g (10⁻² mole) de cyanoborohydrure de sodium (NaBH₃CN) ainsi que 40 ml de méthanol et 0,8 ml d'acide acétique.

On maintient ensuite ce milieu réactionnel sous agitation magnétique dans un bain à 18°C. En cours de réaction, on prélève des parties aliquotes (10µl) du mélange et on les analyse par chromatographie liquide haute performance (CLHP) pour suivre la formation du composé souhaité en même temps que la disparition du (S)-(+)-α-amino-α-(2-chloro-phényl)-acétate de méthyle. Après 3 à 4 heures, la réaction se stabilise et l'analyse montre un rendement de 66% en (+)-(S)-a-(2-thiényl-2-éthylamino)-a-(2-chloro-phényl)-acétate de méthyle.

On ajoute alors 0,96 à 1 g (0,5.10⁻² mole) de 2-thiénylglycidate de sodium 0,3 g (0,5.10⁻² mole) de NaBH₃CN et 0,4 ml d'acide acétique. Après 3 heures de réaction, l'analyse montre un rendement de 98% en composé désiré sous forme de base libre et la disparition de l'ester de départ.

On dilue alors le mélange réactionnel par 250 à 300 ml d'ammoniaque 1 à 2M et on extrait deux fois avec de l'acétate d'éthyle. On rassemble alors les phases organiques, sèche sur sulfate de sodium et évapore. On reprend le résidu dans 40 à 50 ml de méthanol et on traite par 15 à 20 ml (excès) d'acide chlorhydrique 1M dans le méthanol. Après évaporation, le chlorhydrate de (+)-(S)-α-(2-thiényl-2-éthylamino)-α-(2-chloro-phényl)-acétate de méthyle cristallise par addition d'acétone.
Rendement : 2,56 g soit 75%

### EXEMPLE 2

### Chlorhydrate du (+)-(S)-α-(2-thiényl-2-éthylamino)-α-(2-chloro-phényl)-acétate de méthyle

Dans un flacon de 100 ml, on introduit une solution méthanolique 0,25 molaire en 2-thiényl-glycidate de sodium, 0,25 molaire en chiorhydrate de (+)-(S)-α-amino-α-(2-chloro-phényl)-acétate de méthyle, 0,25 molaire en NaBH₃CN et X molaire en acide acétique.

On maintient ensuite ce milieu réactionnel sous agitation magnétique dans un bain à 18°C. En cours de réaction, on prélève des parties aliquotes (10µl) du mélange et on les analyse par CLHP pour suivre la formation du composé souhaité en même temps que la disparition du (+)-(S)-α-amino-α-(2-chlorophényl)-acétate de méthyle. Après 3 à 4 heures, la réaction se stabilise et l'analyse montre les rendements suivants en (+)-(S)-α-(2-thiényl-2-éthylamino)-α-(2-chloro-phényl)-acétate de méthyle calculés sur la conversion de l'ester de départ.

| X (molaire) | Rendement (%) |
|---|---|
| 0 | 50 |
| 0,125 | 52,5 |
| 0,167 | 57,4 |
| 0,330 | 66 |
| 0,420 | 56 |

On poursuit alors la réaction comme décrit à l'Exemple 1 pour obtenir d'abord le (+)-(S)-α-(2-thiényl-2-éthylamino)-α-(2-chloro-phényl)-acétate de méthyle sous forme basique selon des rendements variant de 80 à 98% par CLHP, ensuite le chlorhydrate de ce composé selon des rendements de 60 à 80% après isolement.

### EXEMPLE 3

### (+)-(S)-α-(2-thiényl-2-éthylamino)-α-(2-chloro-phényl)-acétate de méthyle

Ce composé a été préparé selon la méthode décrite à l'Exemple 1 en remplaçant la solution 0,37 molaire en NaBH₃CN (0,9 g dans 40 ml de méthanol) par une solution méthanolique de molarité indiquée ci-dessous, pour obtenir, par CLHP, les rendements suivants en (+)-(S)-α-(2-thiényl-2-éthylamino)-α-(2-chloro-phényl)-acétate de méthyle non isolé.

| Molarité en NaBH₃CN | Rendements (%) |
|---|---|
| 0,22 | 90 |
| 0,30 | 97 |

### EXEMPLE 4

### Chlorohydrate de (+)-(S)-α-(2-thiényl-2-éthylamino)-α-(2-chloro-phényl)-acétate de méthyle

Sous refroidissement (bain d'eau de 14 à 15°C) et sous agitation magnétique, on met en suspendion 1 g de borohydrure de sodium dans 100 ml de dichlorométhane. On ajoute alors lentement en 5 à 10 minutes 4,5 ml d'acide acétique. Dès que la libération d'hydrogène est terminée, on évapore complètement le dichlorométhane à pression réduite et on dissout ensuite le résidu obtenu dans la solution acétique de (+)-(S)-α-amino-α-(2-chlorophényl)-acétate de méthyle obtenue à la Préparation f) ci-dessus. Sous agitation mécanique, on ajoute alors 3 fractions, à savoir 1,54 g ; 1,54 g et 1,34 g de 2-thiénylglycidate de sodium à 5 minutes d'intervalle à une température de 15 à 18°C, puis on laisse réagir durant 20 minutes après la dernière addition.

On dilue ensuite le mélange réactionnel avec 200 ml d'acétate d'éthyle et 400 ml d'eau, puis on ajoute lentement 70 ml d'ammoniaque à 30 %. On agite, décante la phase organique (le pH de la phase aqueuse doit être basique) et on extrait à nouveau la phase aqueuse avec 100 ml d'acétate d'éthyle. On rassemble les phases organiques, lave à l'eau, sèche sur sulfate de sodium et concentre sous vide à pression réduite. On dissout ensuite le résidu obtenu dans 20 ml d'acide chlorhydrique 1 M dans le méthanol et on évapore à nouveau. On reprend le résidu dans 60 ml d'acétone et on maintient à température ambiante jusqu'à formation de cristaux. On ajoute ensuite 100 ml de tertiobutyl méthyl-éther, laisse cristalliser durant 10 à 12 heures en chambre froide puis essore les cristaux formés.

De cette manière, on recueille 2,42 g de chlorhydrate de (+)-(S)-α-amino-α-(2-chloro-phényl)-acétate de méthyle.
Rendement analytique : 95 %
Rendement pondéral : 70 % (l'analyse des eaux mères de cristallisation montre la présence de composé désiré non précipité).

### EXEMPLE 5

### Chlorhydrate de (+)-(S)-α-(2-thiényl-2-éthylamino)-α-(2-chloro-phényl)-acétate de méthyle

A la solution acétique de (+)-(S)-α-amino-α-(2-chloro-phényl)-acétate de méthyle obtenue à la Préparation f) ci-dessus, on ajoute, sous agitation et refroidissement et en 10 minutes environ, 1 g de borohydrure de sodium. On introduit ensuite 4,42 g de 2-thiénylglycidate de sodium en 3 fractions et à une température de 15 à 18°C puis on laisse réagir durant 20 minutes après la dernière addition.

On procède ensuite comme décrit à l'Exemple 4 pour obtenir le chlorhydrate de (+)-(S)-α-(2-thiényl-2-éthylamino)-α-(2-chloro-phényl)-acétate de méthyle.
Rendement analytique : 95 %
Rendement pondéral : 70 % (l'analyse des eaux mères de cristallisation montre la présence de composé désiré non précipité).

## Revendications

1. Procédé pour la préparation de dérivés 2-thiényl-éthylamine de formule générale : 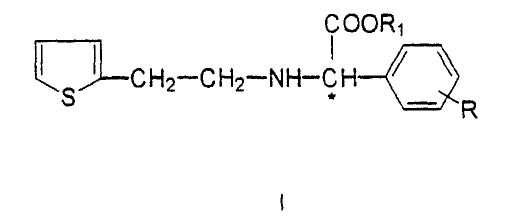 ainsi que leurs sels d'addition d'acide, dans laquelle R représente un atome d'halogène et R₁ représente un groupement alkyle en C₁-C₄, **caractérisé en ce que** l'on fait réagir un dérivé thiénylglycidique de formule générale : 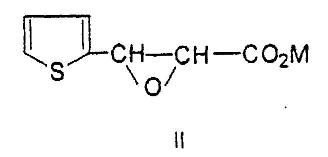 dans laquelle M représente un atome de métal alcalin ou une fraction d'un atome de métal alcalino-terreux, avec un ester de phénylglycine, éventuellement sous forme d'un sel d'acide fort, de formule générale : 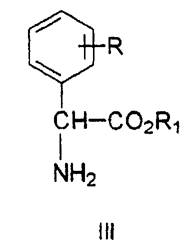 dans laquelle R et R₁ ont la même signification que précédemment, en présence d'un borohydrure de métal alcalin de formule générale :
X - Y IV
dans laquelle X représente un atome de métal alcalin et Y représente un groupement de formule :
- BH₃CN ou -BH _{(4-w)}Z_{w}
dans laquelle Z représente un reste d'acide carboxylique et w représente 1,2 ou 3, ce qui fournit le composé désiré sous forme de base libre que l'on peut traiter si nécessaire, avec un acide pour obtenir un sel d'addition de ce composé.

2. Procédé selon la Revendication 1, **caractérisé en ce que** l'atome d'halogène est situé en position ortho.

3. Procédé selon la Revendication 1, **caractérisé en ce que** l'atome d'halogène est le chlore.

4. Procédé selon une des Revendications 1 à 3, **caractérisé en ce que** R₁ représente méthyle.

5. Procédé selon une des Revendications 1 à 4, **caractérisé en ce que** le dérivé de 2-thiényl-éthylamine de formule I et l'ester de phénylglycine de formule III sont chacun sous forme d'énantiomére (+)-(S).

6. Procédé selon une des Revendications 1 à 5, **caractérisé en ce que** M représente lithium, sodium, potassium, calcium ½ ou magnésium ½.

7. Procédé selon une des Revendications 1 à 6, **caractérisé en ce que** l'ester de phénylglycine de formule III est sous forme d'un sel d'acide fort choisi parmi le chlorhydrate ou le méthanesulfonate.

8. Procédé selon une des Revendications 1 à 7, **caractérisé en ce que** Z représente un reste d'acide carbocylique de formule générale :
R₂-CO₂-
dans laquelle R₂ représente un groupement alkyle en C₁-C₁₀.

9. Procédé selon une des Revendications 1 à 7, **caractérisé en ce que** le borohydrure de métal alcalin correspond au cyanoborohydrure de sodium.

10. Procédé selon la Revendication 9, **caractérisé en ce que** l'on effectue la réaction en présence d'un acide alkylcarboxylique en C₁-C₄.

11. Procédé selon la Revendication 10, **caractérisé en ce que** l'acide carboxylique est utilisé à une concentration n'excédant pas 0,50 mole/l.

12. Procédé selon la Revendication 11, **caractérisé en ce que** l'acide carboxylique est utilisé à une concentration de 0,30 à 0,35 mole/l.

13. Procédé selon une des Revendications 9 à 12, **caractérisé en ce que** l'acide carboxylique est l'acide acétique.

14. Procédé selon une des Revendications 9 à 13, **caractérisé en ce que** l'ester de phénylglycine de formule III est sous forme d'un sel d'acide fort.

15. Procédé selon une des Revendictions 1 à 14, **caractérisé en ce que** le dérivé thiénylglycidique de formule II est utilisé en excès, jusqu'à 0,5 mole supplémentaire par mole d'ester de phénylglycine de formule III.

16. Procédé selon une des Revendications 1 à 7, **caractérisé en ce que** le borohydrure de métal alcalin correspond à un composé de formule IV dans laquelle Y représente le groupement-BH _{(4-w)}Z_{w}.

17. Procédé selon la Revendication 16, **caractérisé en ce que** la réaction est mise en oeuvre dans un acide alkylcarboxylique en C₁-C₄.

18. Procédé selon la Revendication 16 ou 17, **caractérisé en ce que** l'ester de phénylglycine de formule III est utilisé en excès jusqu'à 2,2 équivalents molaires par équivalent molaire de dérivé thiénylglycidique de formule II.

19. Procédé selon une des Revendications 1 à 18, **caractérisé en ce que** le borohydrure de métal alcalin de formule IV est utilisé à une concentration n'excédant pas 0,40 mole/l.

20. Procédé selon une des Revendications 1 à 19, **caractérisé en ce que** l'on prépare le (+)-(S)-α-(2-thiényl-2-éthylamino)-α-(2-chloro-phényl)-acétate de méthyle.

## Patentansprüche

1. Verfahren zur Herstellung von 2-Thienyl-ethylamin-Derivaten der allgemeinen Formel: 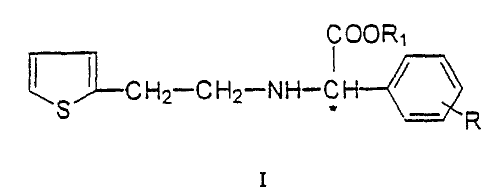 sowie von deren Säureadditionssalzen,worin R ein Halogenatom und R₁ eine C₁-C₄-Alkylgruppe bedeuten, **dadurch gekennzeichnet, daß** man ein Thienylglycid-Derivat der allgemeinen Formel: 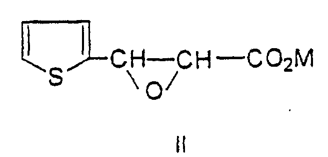 in der M ein Alkalimetallatom oder einen Anteil eines Erdalkalimetallatoms bedeutet, mit einem Phenylglycinester, gegebenenfalls in Form eines Salzes mit einer starken Säure der allgemeinen Formel: 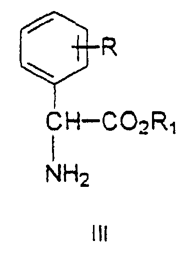 in der R und R₁ die oben angegebenen Bedeutungen besitzen, in Gegenwart eines Alkalimetallborhydrids der allgemeinen Formel:
X - Y IV
in der X ein Alkalimetallatom und Y eine Gruppe der Formel
-BH₃CN oder -BH_{(4-w)}Z_{w}
in der Z den Rest einer Carbonsäure und w 1, 2 oder 3 darstellen, bedeuten, umsetzt, so daß man die gewünschte Verbindung in Form der freien Base erhält, welche man erforderlichenfalls mit einer Säure zur Bildung eines Additionssalzes dieser Verbindung umsetzen kann.

2. Verfahren nach Anspruch 1, **dadurch gekennzeichnet, daß** das Halogenatom in der ortho-Stellung steht.

3. Verfahren nach Anspruch 1, **dadurch gekennzeichnet, daß** das Halogenatom Chlor bedeutet.

4. Verfahren nach einem der Ansprüche 1 bis 3, **dadurch gekennzeichnet, daß** R₁ Methyl bedeutet.

5. Verfahren nach einem der Ansprüche 1 bis 4, **dadurch gekennzeichnet, daß** das 2-Thienyl-ethylamin-Derivat der Formel I und der Phenylglycinester der Formel III jeweils in Form des (+)-(S)-Enantiomeren vorliegen.

6. Verfahren nach einem der Ansprüche 1 bis 5, **dadurch gekennzeichnet, daß** M Lithium, Natrium, Kalium, ½ Calcium oder ½ Magnesium bedeutet.

7. Verfahren nach einem der Ansprüche 1 bis 6, **dadurch gekennzeichnet, daß** der Phenylglycinester der Formel III in Form eines Salzes mit einer starken Säure ausgewählt aus dem Hydrochlorid und dem Methansulfonat vorliegt.

8. Verfahren nach einem der Ansprüche 1 bis 7, **dadurch gekennzeichnet, daß** Z den Rest einer Carbonsäure der allgemeinen Formel:
R₂-CO₂-
in der R₂ eine C₁-C₁₀-Alkylgruppe darstellt, bedeutet.

9. Verfahren nach einem der Ansprüche 1 bis 7, **dadurch gekennzeichnet, daß** das Alkalimetallborhydrid Natriumcyanborhydrid ist.

10. Verfahren nach Anspruch 9, **dadurch gekennzeichnet, daß** man die Reaktion in Gegenwart einer C₁-C₄-Alkylcarbonsäure durchführt.

11. Verfahren nach Anspruch 10, **dadurch gekennzeichnet, daß** die Carbonsäure in einer Konzentration von nicht mehr als 0,50 Mol/l eingesetzt wird.

12. Verfahren nach Anspruch 11, **dadurch gekennzeichnet, daß** die Carbonsäure in einer Konzentration von 0,30 bis 0,35 Mol/l eingesetzt wird.

13. Verfahren nach einem der Ansprüche 9 bis 12, **dadurch gekennzeichnet, daß** die Carbonsäure Essigsäure ist.

14. Verfahren nach einem der Ansprüche 9 bis 13, **dadurch gekennzeichnet, daß** der Phenylglycinester der Formel III in Form eines Salzes mit einer starken Säure eingesetzt wird.

15. Verfahren nach einem der Ansprüche 1 bis 14, **dadurch gekennzeichnet, daß** das Thienylglycid-Derivat der Formel II in einem Überschuß von 0,5 Mol pro Mol des Phenylglycinesters der Formel III eingesetzt wird.

16. Verfahren nach einem der Ansprüche 1 bis 7, **dadurch gekennzeichnet, daß** das Alkalimetallborhydrid einer Verbindung der Formel IV entspricht, in der Y die Gruppe -BH_{(4-w)}Z_{w} bedeutet.

17. Verfahren nach Anspruch 16, **dadurch gekennzeichnet, daß** die Reaktion in einer C₁-C₄-Alkylcarbonsäure durchgeführt wird.

18. Verfahren nach Anspruch 16 oder 17, **dadurch gekennzeichnet, daß** der Phenylglycinester der Formel III in einem Überschuß bis zu 2,2 Moläquivalenten pro Moläquivalent des Thienylglycid-Derivats der Formel II eingesetzt wird.

19. Verfahren nach einem der Ansprüche 1 bis 18, **dadurch gekennzeichnet, daß** das Alkalimetallborhydrid der Formel IV in einer Konzentration von nicht mehr als 0,40 Mol/l eingesetzt wird.

20. Verfahren nach einem der Ansprüche 1 bis 19, **dadurch gekennzeichnet, daß** man den (+)-(S)-α-(2-Thienyl-2-ethylamlno)-α-(2-chlorphenyl)-essigsäuremethylester herstellt.

## Claims

1. Process for preparing 2-thienyl-ethylamine derivatives of general formula: 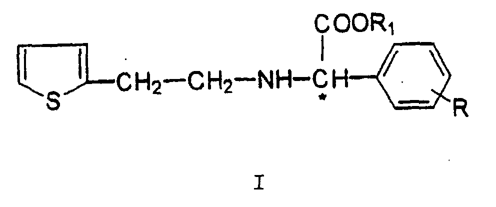 and the acid addition salts thereof, wherein R denotes a halogen atom and R₁ denotes a C₁-C₄-alkyl group, **characterised in that** a thienylglycide derivative of general formula: 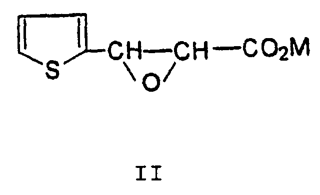 wherein M denotes an alkali metal atom or a fraction of an alkaline earth metal atom, is reacted with a phenylglycine ester, optionally in the form of a strong acid salt, of general formula: 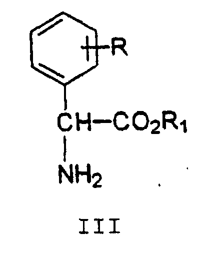 wherein R and R₁ have the meanings given hereinbefore, in the presence of an alkali metal borohydride of general formula:
X - Y IV
wherein X denotes an alkali metal atom and Y denotes a group of formula:
- BH₃CN or -BH_{(4-w)}Z_{w}
wherein Z denotes a carboxylic acid group and w denotes 1, 2 or 3, yielding the desired compound in the form of a free base which may if necessary be treated with an acid to obtain an addition salt of this compound.

2. Process according to claim 1, **characterised in that** the halogen atom is in the ortho position.

3. Process according to claim 1, **characterised in that** the halogen atom is chlorine.

4. Process according to one of claims 1 to 3, **characterised in that** R₁ denotes methyl.

5. Process according to one of claims 1 to 4, **characterised in that** the 2-thienyl-ethylamine derivative of formula I and the phenylglycine ester of formula III are both in the form of an (+)-(S) enantiomer.

6. Process according to one of claims 1 to 5, **characterised in that** M denotes lithium, sodium, potassium, % calcium or % magnesium.

7. Process according to one of claims 1 to 6, **characterised in that** the phenylglycine ester of formula III is in the form of a strong acid salt selected from the hydrochloride or the methanesulphonate.

8. Process according to one of claims 1 to 7, **characterised in that** Z represents a carbocyclic acid group of general formula:
R₂-CO₂-
wherein R₂ denotes a C₁-C₁₀-alkyl group.

9. Process according to one of claims 1 to 7, **characterised in that** the alkali metal borohydride corresponds to sodium cyanoborohydride.

10. Process according to claim 9, **characterised in that** the reaction is carried out in the presence of a C₁-C₄-alkylcarboxylic acid.

11. Process according to claim 10, **characterised in that** the carboxylic acid is used in a concentration not exceeding 0.50 mol/l.

12. Process according to claim 11, **characterised in that** the carboxylic acid is used in a concentration of 0.30 to 0.35 mol/l.

13. Process according to one of claims 9 to 12, **characterised in that** the carboxylic acid is acetic acid.

14. Process according to one of claims 9 to 13, **characterised in that** the phenylglycine ester of formula III is in the form of a strong acid salt.

15. Process according to one of claims 1 to 14, **characterised in that** the thienylglycide derivative of formula II is used in excess, up to 0.5 additional mol per mol of the phenylglycine ester of formula III.

16. Process according to one of claims 1 to 7, **characterised in that** the alkali metal borohydride corresponds to a compound of formula IV wherein Y represents the group -BH_{(4-w)}Z_{w}.

17. Process according to claim 16, **characterised in that** the reaction is carried out in a C₁-C₄-alkylcarboxylic acid.

18. Process according to claim 16 or 17, **characterised in that** the phenylglycine ester of formula III is used in an excess of up to 2.2 molar equivalents per molar equivalent of thienylglycide derivative of formula II.

19. Process according to one of claims 1 to 18, **characterised in that** the alkali metal borohydride of formula IV is used in a concentration not exceeding 0.40 mol/l.

20. Process according to one of claims 1 to 19, **characterised in that** methyl (+)-(S)-α-(2-thienyl-2-ethylamino)-α-(2-chlorophenyl)-acetate is prepared.
